# EUROPEAN PATENT APPLICATION

(11) **EP 1 419 775 A1**
(43) Date of publication of application: **19.05.2004**
(21) Application number: 02751749.9
(22) Date of filing: 29.07.2002
(51) Int. Cl.: A61K 31/495, A61K 31/551, A61K 9/48, A61K 47/02, A61P 9/04

(54) **PHARMACEUTICAL PREPARATIONS CONTAINING AMINOBENZENE-SULFONIC ACID DERIVATIVES AS THE ACTIVE INGREDIENT**

(30) Priority: 30.07.2001 JP 2001229086
(71) Applicant: Mitsubishi Pharma Corporation, Osaka-shi, Osaka 541-0046 (JP)
(72) Inventor: INAMORI,T. MITSUBISHI PHARMA CORP.Tokyo Head Offi., Chuo-ku, Tokyo 103-8405 (JP); MIURA, Susumu, Nerima-ku, Tokyo 176-0002 (JP)
(74) Representative: HOFFMANN EITLE
(86) International application number: PCT/JP2002/007656
(87) International publication number: WO 2003/011296

(57) **Abstract**

A stable pharmaceutical preparation containing an aminobenzenesulfonic acid derivative represented by the following formula (I) known as a therapeutic agent for cardiac insufficiency: 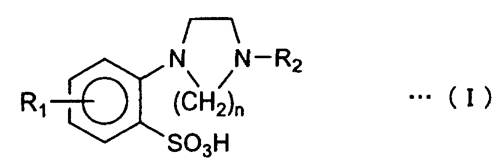 or a salt thereof, or a hydrate thereof or a solvate thereof as an active ingredient, wherein each production of substance A having a retention time of about 6.4 minutes in a high performance liquid chromatography, substance B having a retention time of about 15.6 minutes in the high performance liquid chromatography, and substance C having about 22.8 minutes in the high performance liquid chromatography is substantially suppressed, wherein said high performance liquid chromatography is performed at a controlled flow rate for elution so as to give a retention time of about 7 minutes of said active ingredient by using an ultraviolet absorptiometer at 220 nm, an octylsilylated silica gel packed column (4 mm x 250 mm) at 40°C, and a mobile phase prepared by dissolving 7.8 g of sodium dihydrogenphosphate dihydrate in 1000 mL of a mixture of water/acetonitrile (6:1) after said pharmaceutical preparation is extracted with a diluent prepared by dissolving 7.8 g of sodium dihydrogenphosphate dihydrate in 1000 mL of a mixture of water/acetonitrile (6:1) and adjusting a pH to 7.0 with addition of 8 mol/L sodium hydroxide solution.

## Description

### Technical Field

The present invention relates to a pharmaceutical preparation which is coated for prevention of the formation of photodecomposition products.

### Background Art

As methods for shielding lights for prevention of photodecomposition of pharmaceutical preparations, known methods include light-shielding coatings which shade a bulk of a drug itself, or means for shielding lights with packages such as light-shielding PTP packages, double-sided aluminum packages, light-shielding paper and the like. Among them, the methods of shielding lights with packages cannot guarantee stability after opening of the packages, and therefore it is considered that the method of shielding the bulk of a drug itself from lights is desirable. As a light-shielding ingredient in the light-shielding coatings, titanium oxide is commonly used,, and variety of preparations are known.

The aminobenzenesulfonic acid derivatives described in Japanese Patent Unexamined Publication (Kokai) Nos. 3-7263, 4-139127, 9-221479, 10-298077 and WO99/40919 are known as medicaments for treatment of cardiac insufficiency. These patent publications disclose general preparation methods and pharmaceutical formulations. When these compounds are made into tablets, light-shielding coatings are required, since plain tablets are found to generate photodecomposition products in a light irradiation test. However, when generally used titanium oxide is used as an ingredient of a light-shielding coating, the aforementioned aminobenzenesulfonic acid derivatives react with the titanium oxide to generate other photodecomposition products, and therefore sufficient stability cannot be guaranteed by ordinary light-shielding coatings.

Therefore, an object of the present invention is to provide stable drugs comprising the aforementioned aminobenzenesulfonic acid derivatives.

### Disclosure of the Invention

The inventors of the present invention conducted various researches to achieve the foregoing object. As a result, they found that, by first providing a coating that is free from titanium oxide on plain tablets comprising the aforementioned aminobenzenesulfonic acid derivative and then providing a coating containing titanium oxide, tablets can be provided which successfully prevent production of a decomposition product resulting from the reaction of the aminobenzenesulfonic acid derivative as the bulk drug with light, as well as production of a decomposition product resulting from the reaction of the aminobenzenesulfonic acid as the bulk drug, titanium oxide, and light. The present invention was thus achieved.

The gist of the present invention resides in a pharmaceutical preparation containing an aminobenzenesulfonic acid derivative represented by the following formula (I): 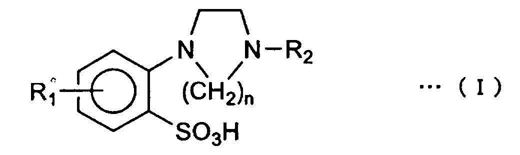 wherein R₁ represents hydrogen atom, a C₁-C₆ alkyl group, a C₃-C₇ cycloalkyl group, a halogenated C₁-C₄ alkyl group, a halogen atom or a C₆-C₁₂ aryl group; R₂ represents hydrogen atom, a C₁-C₆ alkyl group or a C₇-C₁₂ aralkyl group which may have one or more substituents selected from the group consisting of cyano group, nitro group, a C₁-C₆ alkoxyl group, a halogen atom, a C₁-C₆ alkyl group and an amino group; and n represents an integer of 1 to 4 or a salt thereof, or a hydrate thereof or a solvate thereof as an active ingredient, wherein each production of substance A having a retention time of about 6.4 minutes in a high performance liquid chromatography, substance B having a retention time of about 15.6 minutes in the high performance liquid chromatography, and substance C having about 22.8 minutes in the high performance liquid chromatography is substantially suppressed, wherein said high performance liquid chromatography is performed at a controlled flow rate for elution so as to give a retention time of about 7 minutes of said active ingredient by using an ultraviolet absorptiometer at 220 nm, an octylsilylated silica gel packed column (4 mm x 250 mm) at 40°C, and a mobile phase prepared by dissolving 7.8 g of sodium dihydrogenphosphate dihydrate in 1000 mL of a mixture of water/acetonitrile (6:1) after said pharmaceutical preparation is extracted with a diluent prepared by dissolving 7.8 g of sodium dihydrogenphosphate dihydrate in 1000 mL of a mixture of water/acetonitrile (6:1) and adjusting a pH to 7.0 with addition of 8 mol/L sodium hydroxide solution.

Preferred embodiments of the present invention include the aforementioned pharmaceutical preparation, characterized in that the coating layer consists of two layers; the aforementioned pharmaceutical preparation, characterized in that the coating layer consists of a first coating layer for substantially suppressing production of the substance A, and a second coating layer for substantially suppressing production of the substances B and C; the aforementioned pharmaceutical preparation, characterized in that the first coating layer does not contain titanium oxide; and the aforementioned pharmaceutical preparation, characterized in that the second coating layer contains titanium oxide; the pharmaceutical preparation in the form of a tablet; and the pharmaceutical preparation, wherein a plain tablet is coated with the first coating layer and then coated with the second coating layer.

Another gist of the present invention resides in a tablet wherein a plain tablet containing an aminobenzenesulfonic acid derivative represented by the following formula (I): 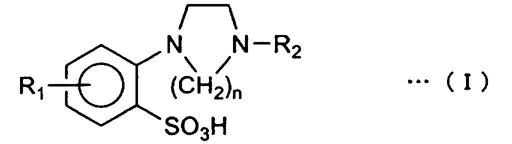 wherein R₁ represents hydrogen atom, a C₁-C₆ alkyl group, a C₃-C₇ cycloalkyl group, a halogenated C₁-C₄ alkyl group, a halogen atom or a C₆-C₁₂ aryl group; R₂ represents hydrogen atom, a C₁-C₆ alkyl group or a C₇-C₁₂ aralkyl group which may have one or more substituents selected from the group consisting of cyano group, nitro group, a C₁-C₆ alkoxyl group, a halogen atom, a C₁-C₆ alkyl group and an amino group; and n represents an integer of 1 to 4, or a salt thereof, or a hydrate thereof or solvate thereof as an active ingredient, is applied with two coating layers, which is characterized to comprise a first coating layer having a property of substantially suppressing production of a substance produced from a reaction of said active ingredient, titanium oxide, and light, and a second coating layer having a property of substantially suppressing production of a substance produced from a reaction of said active ingredient and light.

Preferred embodiments of the aforementioned tablets include those wherein the first coating layer does not contain titanium oxide; those wherein the second coating layer contains titanium oxide; and those wherein the plain tablet is coated with the first coating layer and then coated with the second coating layer.

### Best Mode for Carrying out the Invention

The present invention will be explained in detail below.

The active ingredient of the pharmaceutical preparation of the present invention includes the aminobenzenesulfonic acid derivatives represented by the aforementioned formula (I) or salts thereof, or hydrates thereof or solvates thereof. In the aforementioned formula (I), examples of the C₁-C₆ alkyl group defined by R₁ include, for example, methyl group, ethyl group, propyl group, isopropyl group, butyl group, isobutyl group, sec-butyl group, tert-butyl group, pentyl group, isopentyl group, neopentyl group, tert-pentyl group, hexyl group, isohexyl group and the like. Examples of the C₃-C₇ cycloalkyl group include cyclopropyl group, cyclobutyl group, cyclopentyl group, cyclohexyl group, cycloheptyl group and the like. Examples of the halogenated C₁-C₄ alkyl group include, for example, trifluoromethyl group, trifluoroethyl group, pentafluoroethyl group and the like. Examples of the halogen atom include, for example, fluorine atom, chlorine atom, bromine atom and the like. Examples of the C₆-C₁₂ aryl group include, for example, phenyl group, naphthyl group and the like.

Preferred examples of R₁ include hydrogen atom, a C₁-C₆ alkyl group, a C₅-C₆ cycloalkyl group, trifluoromethyl group, a halogen atom and phenyl group, and more preferred examples of R₁ are a C₁-C₃ alkyl group, cyclohexyl group, trifluoromethyl group, chlorine atom, bromine atom and phenyl group. Particularly preferred are methyl group and propyl group.

Examples of the C₁-C₆ alkyl group defined by R₂ include, for example, the alkyl groups mentioned above as R₁. Examples of the C₇-C₁₂ aralkyl group include, for example, benzyl group, phenethyl group, naphthylmethyl group and the like. This aralkyl group may have one or more substituents selected from the group consisting of cyano group; nitro group; a C₁-C₆ alkoxy group such as methoxy group, ethoxy group, propoxy group, isopropoxy group, butoxy group, isobutoxy group, tert-butoxy group, pentyloxy group, isopentyloxy group, tert-pentyloxy group and hexyloxy group; a halogen atom as defined above for R₁; an alkyl group as defined above for R₁ and an amino group.

Preferred examples of R₂ include hydrogen atom, a C₁-C₃ alkyl group and a C₇-C₁₂ aralkyl group which may have one or more substituents selected from a C₁-C₃ alkyl group, a C₁-C₃ alkoxyl group and a halogen atom, and more preferred examples of R₂ include hydrogen atom and a C₇-C₁₂ aralkyl group which may have one or more substituents selected from C₁-C₃ alkoxyl groups. Particularly preferred is hydrogen atom.

In the aforementioned formula (I), n is preferably 2.

Specific examples of the compounds suitably used for the present invention include the compounds shown in Tables 1 and 2 set out below.

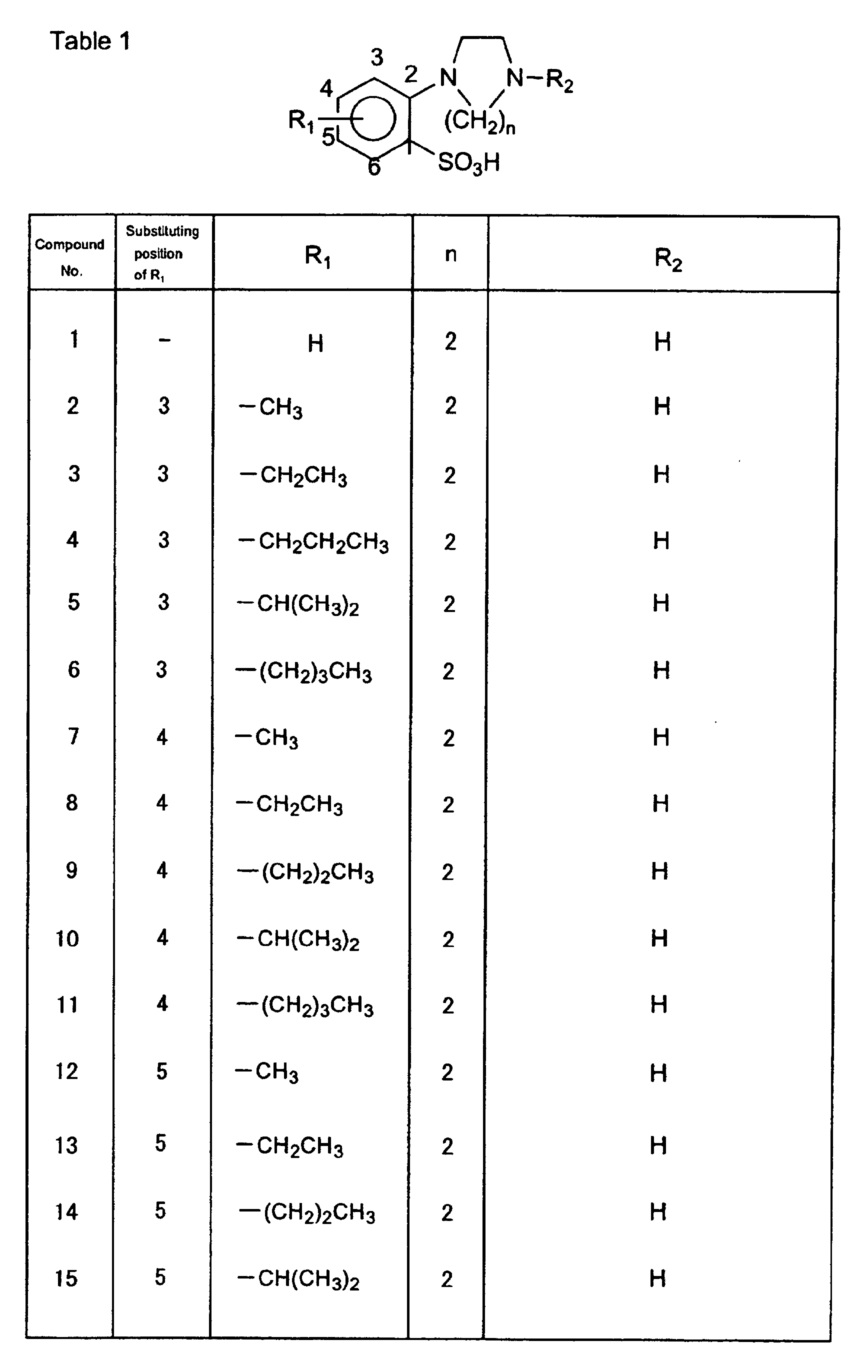

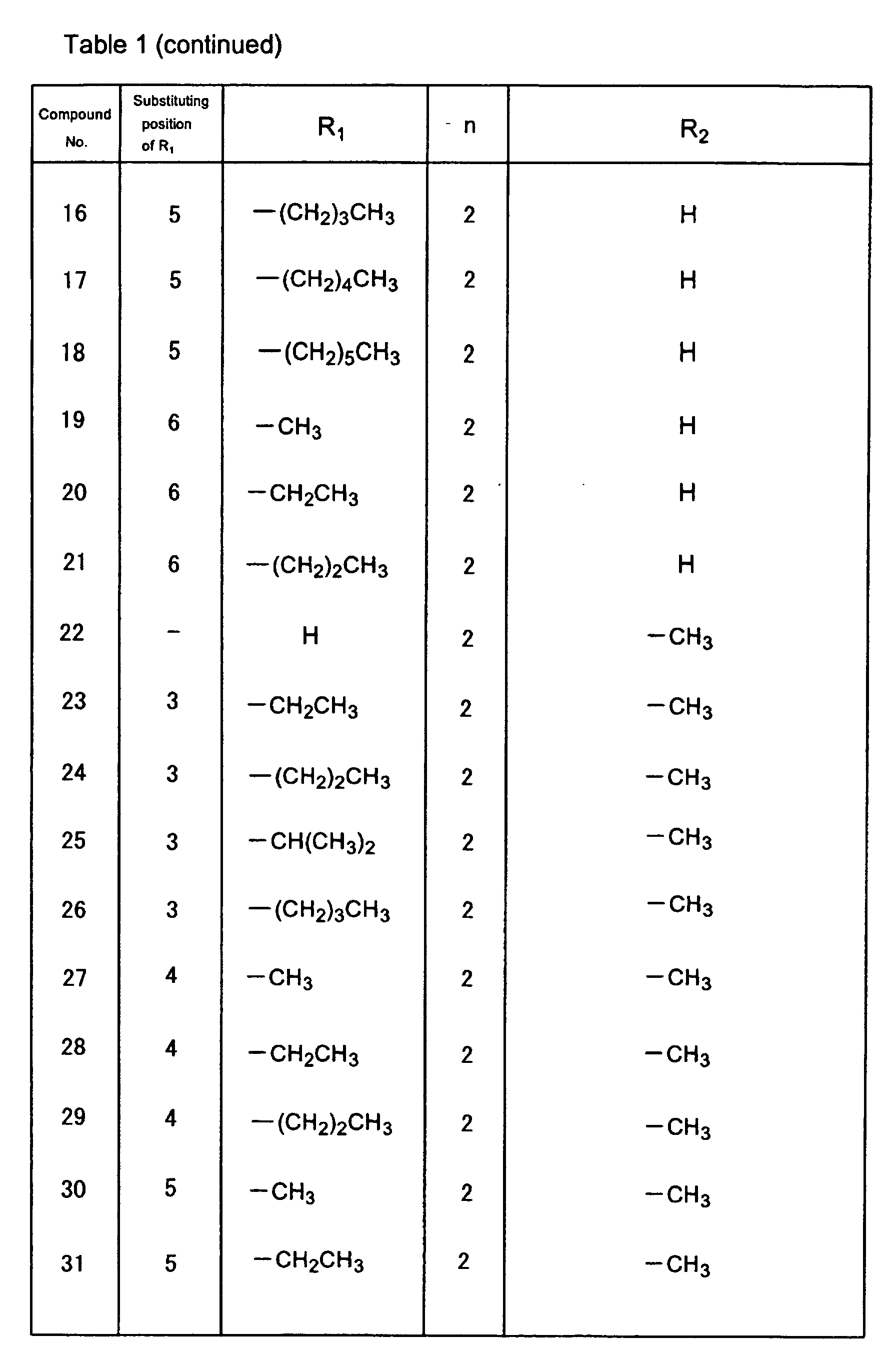

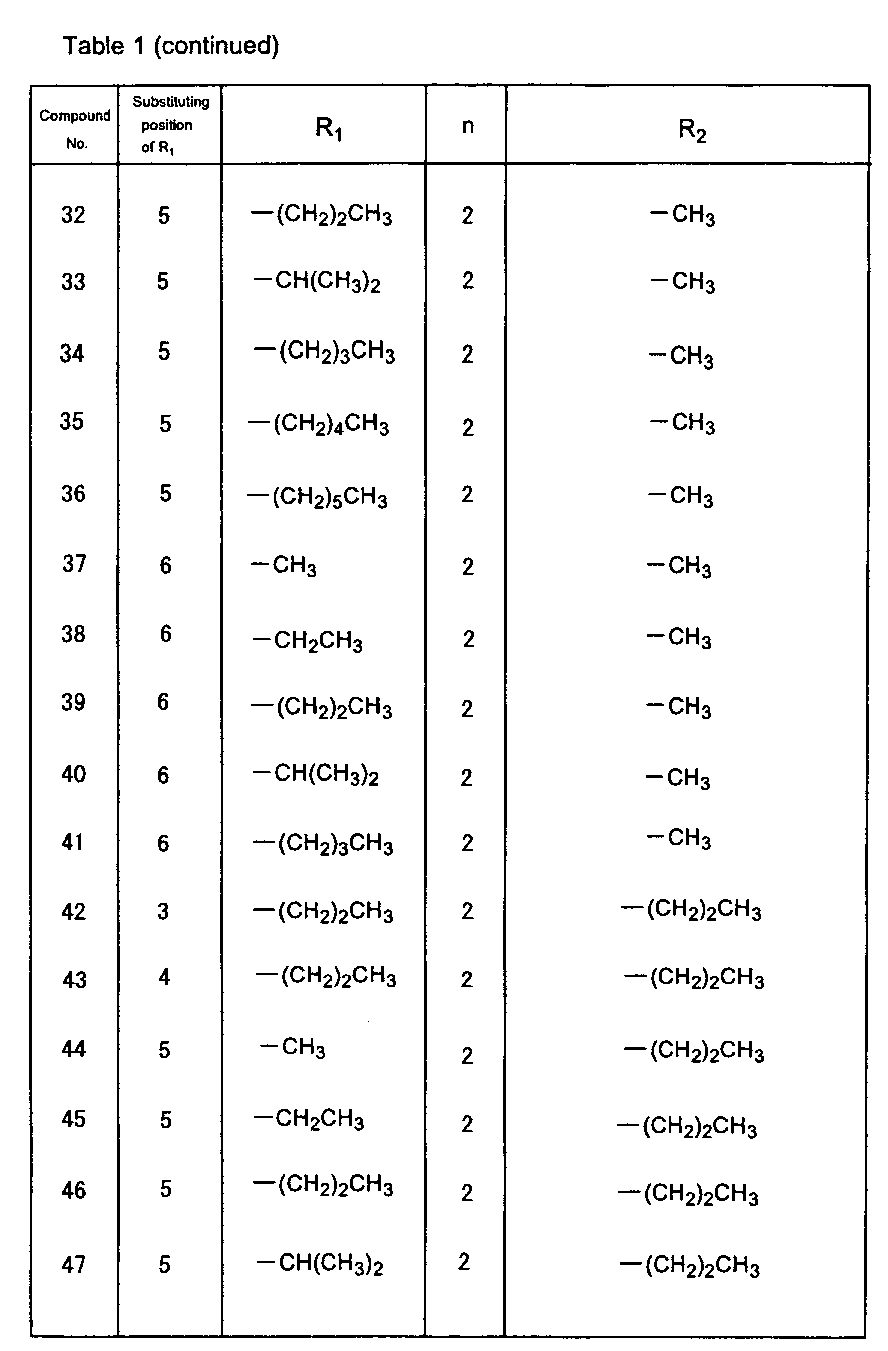

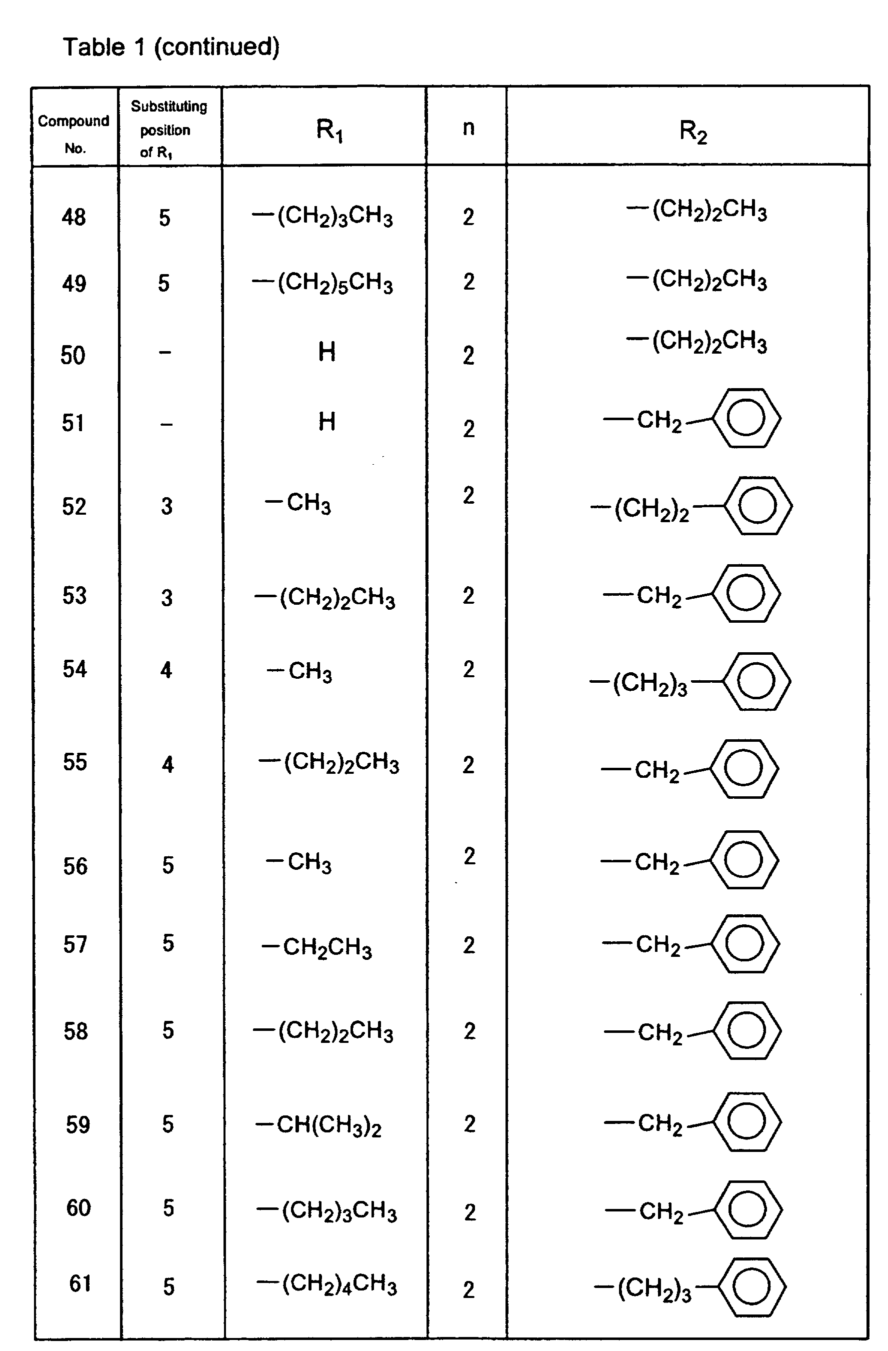

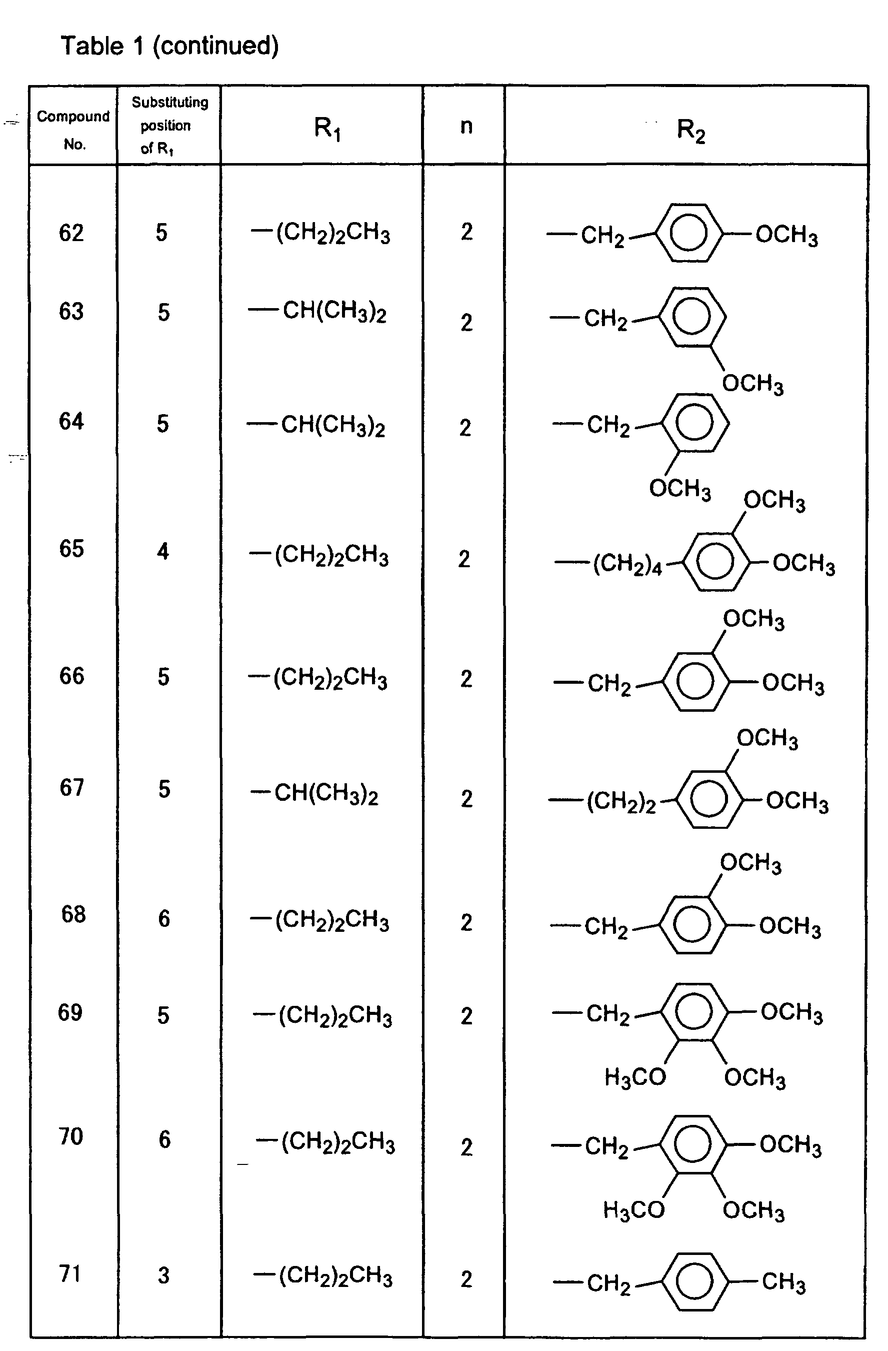

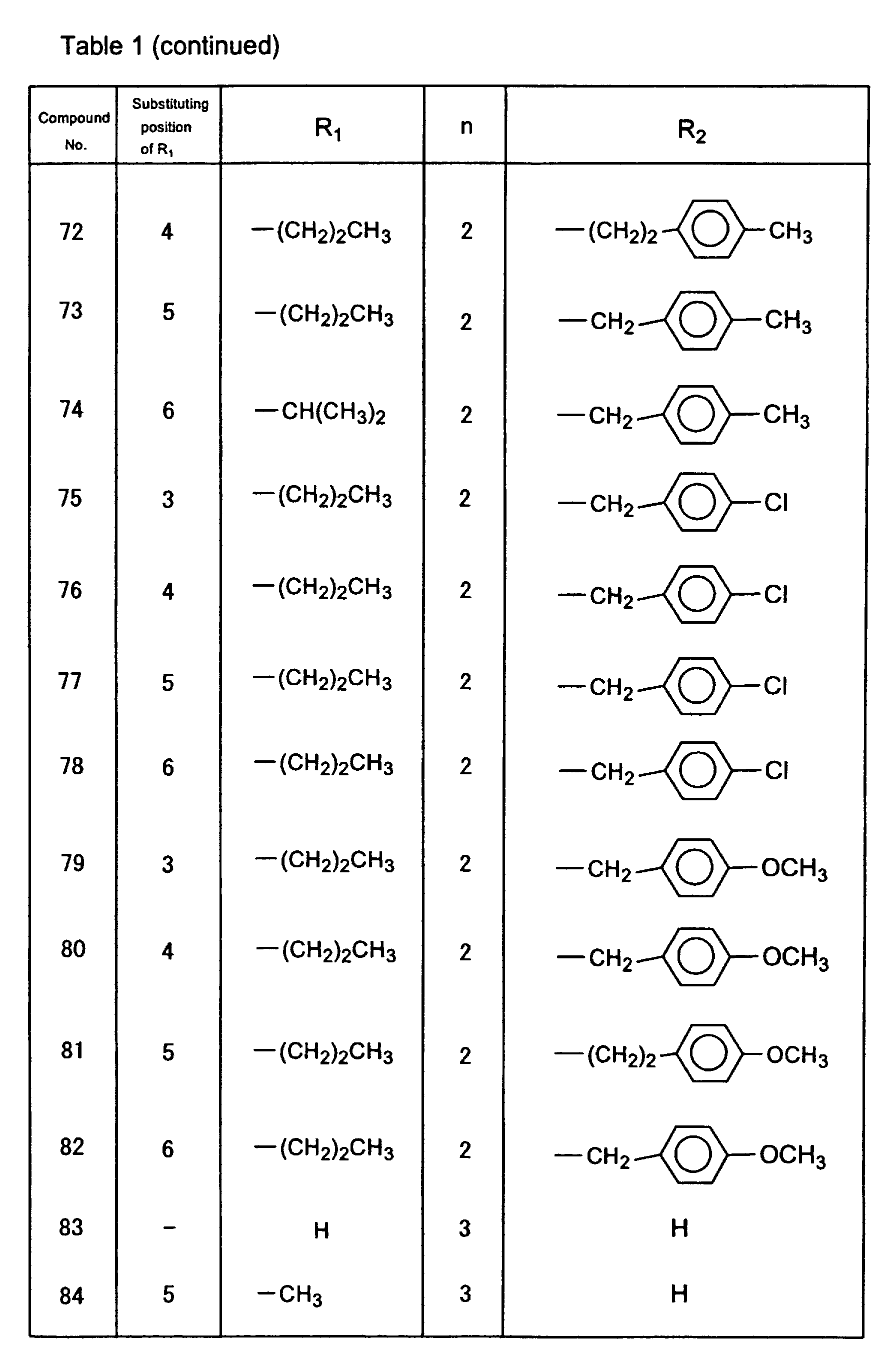

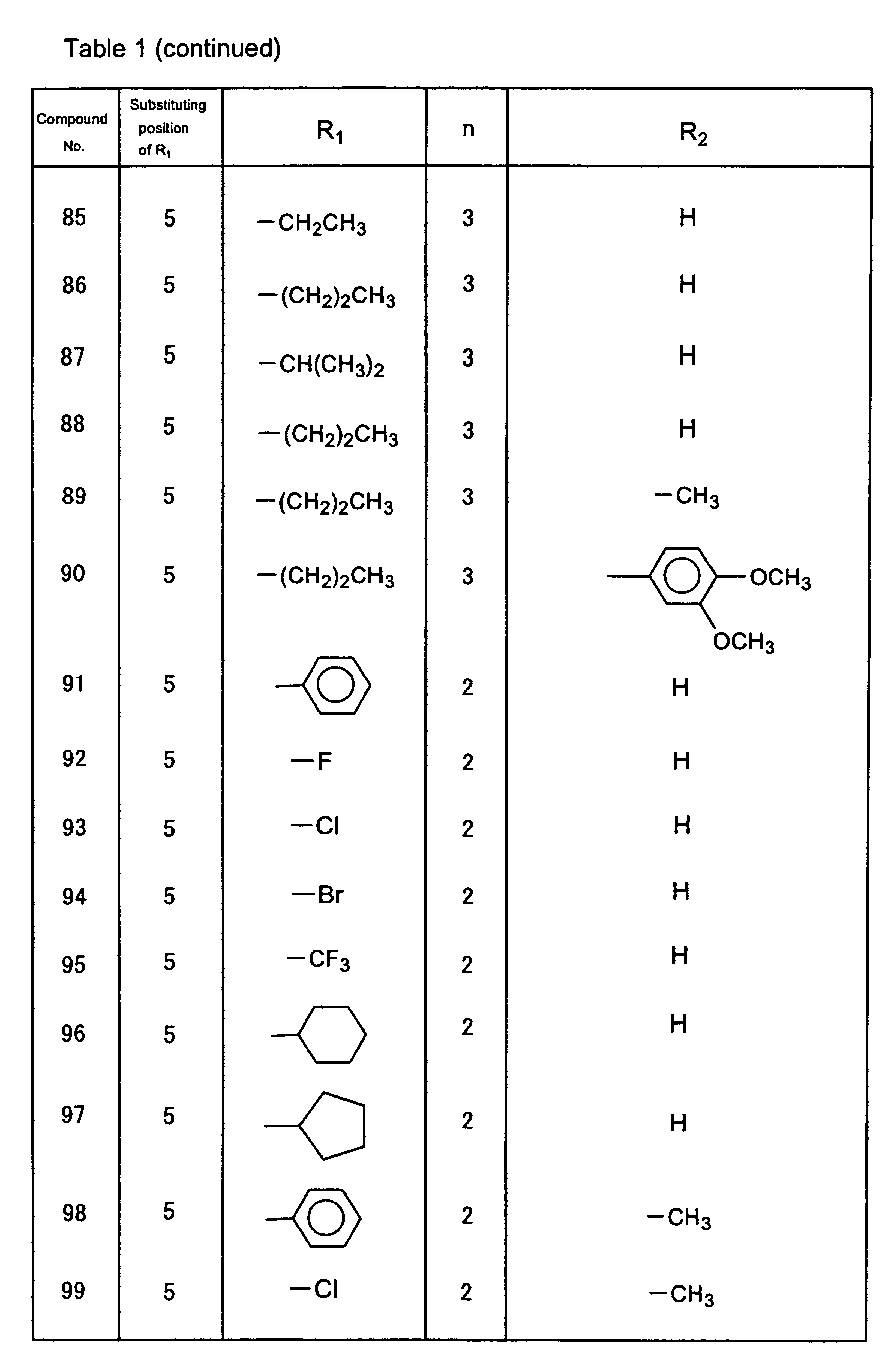

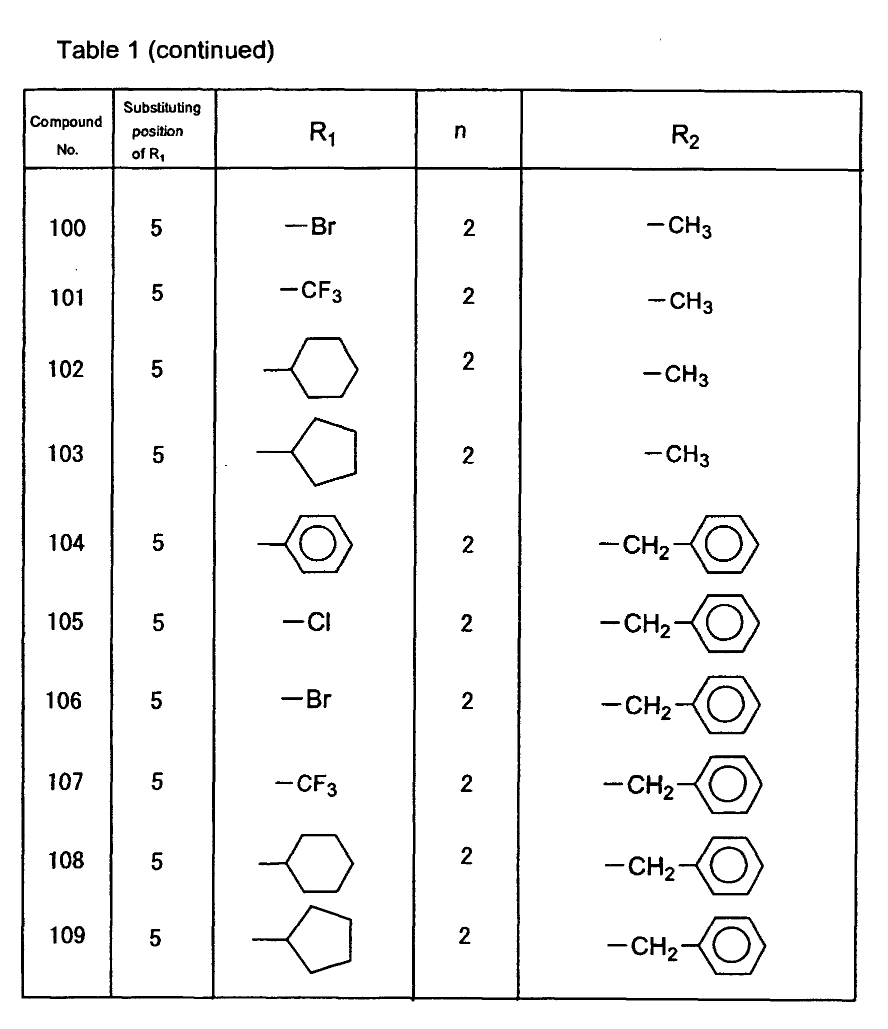

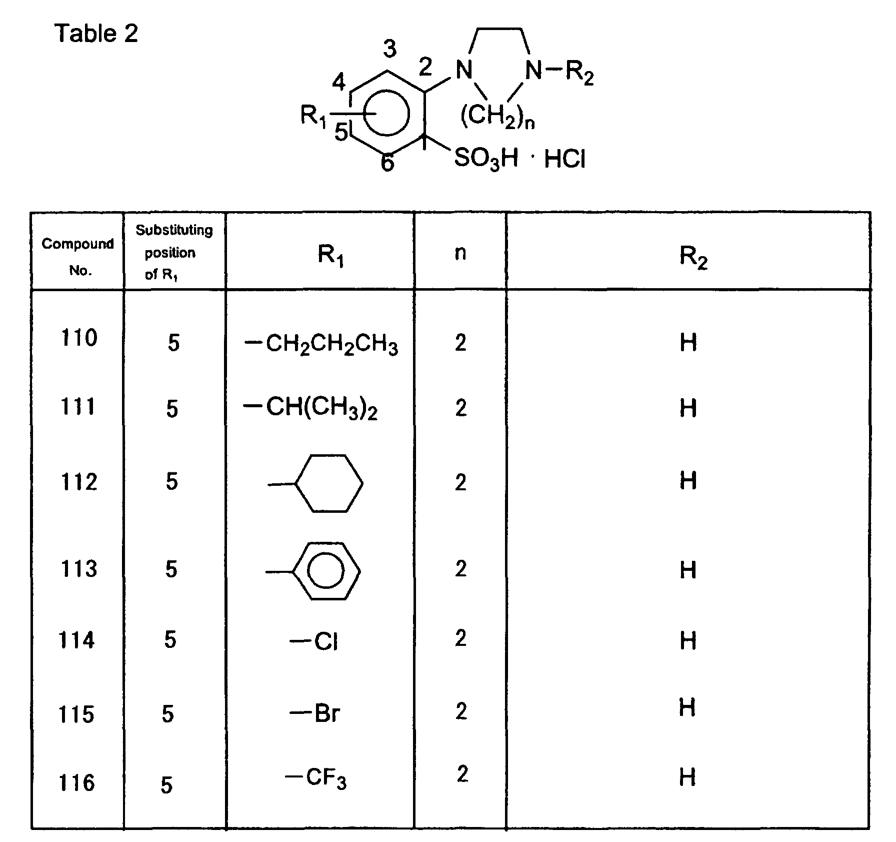

Among the compounds shown in Tables 1 and 2 set out above, the compounds wherein the substitution of R₁ is in the 5-position are preferred, and further preferred compounds include the following compounds:
5-methyl-2-(1-piperazinyl)benzenesulfonic acid;
5-trifluoromethyl-2-(1-piperazinyl)benzenesulfonic acid;
5-n-propyl-2-(1-piperazinyl)benzenesulfonic acid;
5-phenyl-2-(1-piperazinyl)benzenesulfonic acid;
5-chloro-2-(1-piperazinyl)benzenesulfonic acid;
5-bromo-2-(1-piperazinyl)benzenesulfonic acid;
5-iso-propyl-2-(1-piperazinyl)benzenesulfonic acid;
5-cyclohexyl-2-(1-piperazinyl)benzenesulfonic acid;
5-n-propyl-2-(1-homopiperazinyl)benzenesulfonic acid;
5-n-propyl-2-[4-(2,3,4-trimethoxybenzyl)-1-piperazinyl]benzenesulfonic acid;
5-n-propyl-2-[4-(3,4-dimethoxybenzyl)-1-piperazinyl]benzenesulfonic acid.

Among the aforementioned compounds, particularly preferred examples include 5-methyl-2-(1-piperazinyl)benzenesulfonic acid and 5-n-propyl-2-(1-piperazinyl)benzenesulfonic acid.

Pharmacologically acceptable salts of the compounds mentioned above also fall within the scope of the present invention. Examples of the salts of the aforementioned compounds include, for example, alkali metal salts and alkaline earth metal salts such as sodium salts, potassium salts, magnesium salts, calcium salts and aluminum salts; amine salts, for example, ammonium salts, lower alkylamine salts such as triethylamine salts, hydroxy(lower alkyl)amine salts such as 2-hydroxyethylamine salts, bis(2-hydroxyethyl)amine salts, tris(hydroxymethyl)aminomethane salts and N-methyl-D-glucamine salts, cycloalkylamine salts such as dicyclohexylamine salts, benzylamine salts and dibenzylamine salts such as N,N-dibenzylethylenediamine salts; inorganic acid salts such as hydrochlorides, hydrobromides, sulfates and phosphates; organic acid salts such as fumarates, succinates, oxalates and lactates and the like.

Besides the compounds in the forms of salts or free forms, any hydrates and solvates thereof may also be used as the active ingredient of the pharmaceutical preparation of the present invention. Examples of solvents that can form solvates of the aforementioned compounds include, for example, methanol, ethanol, isopropyl alcohol, acetone, ethyl acetate, methylene chloride and the like.

A most preferred example of the active ingredient according to the present invention includes 5-methyl-2-(1-piperazinyl)benzenesulfonic acid monohydrate.

The aminobenzenesulfonic acid derivatives represented by the aforementioned formula (I) are known compounds, and they are easily synthesizable and readily available to those skilled in the art by the methods described in, for example, Japanese Patent Unexamined Publication (KOKAI) Nos. (Hei)3-7263 and (Hei)9-221479, European Patent Publication Nos. 390654 and 779283, U.S. Patent Nos. 5,053,409 and 5,990,113 and the like.

The present invention will be explained by referring to a tablet as a representative example of the pharmaceutical preparation of the present invention. However, the pharmaceutical preparation of the present invention is not limited to tablets unless a preparation is beyond the scope of the present invention.

For preparation of tablets using the aforementioned active ingredient, ordinary formulation techniques are used. The above compound is mixed with an excipient, granulated and sized as required, and then mixed with a lubricant and compressed. Examples of the excipient include lactose, mannitol, cornstarch, potato starch, crystalline cellulose and the like. Examples of a binder used for the granulation include hydroxypropylcellulose, polyvinyl alcohol, povidone and the like. Examples of the lubricant include magnesium stearate, calcium stearate, stearic acid and the like. If necessary, a disintegrating agent, a flowability improver and the like may be added. Examples of the disintegrating agent include carboxymethyl starch sodium, croscarmellose sodium, carmellose calcium, crospovidone and the like. Examples of the flowability improver include water-containing silicon dioxide, light anhydrous silicic acid and the like.

The amounts of these additives are determined from viewpoints of dosage form design, reservation of stability of the preparation, suitability for scaling up of manufacture and the like.

For mixing, an ordinary vertical granulator or the like is used. When granulation is carried out, an ordinary granulation method may be applied for the granulation. Examples include fluidized bed granulation method, agitation granulation method and the like. A fluid bed granulator, vertical granulator and the like are used, respectively. When a lubricant is mixed, a V-shaped rotary mixer or the like is used. For compression, an ordinary tabletting machine is used.

According to the present invention, a coating is provided on a plain tablet comprising the compound obtained above as an active ingredient, so that each production of substance A having a retention time of about 6.4 minutes in a high performance liquid chromatography, substance B having a retention time of about 15.6 minutes in the high performance liquid chromatography, and substance C having about 22.8 minutes in the high performance liquid chromatography is substantially suppressed, wherein said high performance liquid chromatography is performed at a controlled flow rate for elution so as to give a retention time of about 7 minutes of said active ingredient by using an ultraviolet absorptiometer at 220 nm, an octylsilylated silica gel packed column (4 mm x 250 mm) at 40°C, and a mobile phase prepared by dissolving 7.8 g of sodium dihydrogenphosphate dihydrate in 1000 mL of a mixture of water/acetonitrile (6:1) after said pharmaceutical preparation as a final product is extracted with a diluent prepared by dissolving 7.8 g of sodium dihydrogenphosphate dihydrate in 1000 mL of a mixture of water/acetonitrile (6:1) and adjusting a pH to 7.0 with addition of 8 mol/L sodium hydroxide solution.

The coating layer preferably consists of two of layers. In particular, the coating layer is preferably formulated so that production of the substance A can be substantially suppressed by a first coating layer, and production of the substances B and C can be substantially suppressed by a second coating layer.

The first coating layer is not particularly limited so long as the layer is formulated so as to substantially suppress the production of the substance A. An example includes a coating layer which does not contain titanium oxide. So long as the layer is free from titanium oxide, its formulation for other ingredients is not particularly limited. It is sufficient that the first coating layer has such a formulation that the production of the substance A, which is produced by a reaction of the active ingredient, titanium oxide, and light, can be substantially suppressed.

The second coating layer is not particularly limited so long as the layer is formulated so as to substantially suppress the production of the substances B and C. An example includes a coating layer which contains titanium oxide. So long as the layer contains titanium oxide, its formulation for other ingredients is not particularly limited. It is sufficient that the second coating layer has such a formulation that the production of the substances B and C, which are produced by a reaction of the active ingredient and light, can be substantially suppressed.

In the above descriptions, an example using titanium oxide as a light-shielding agent is explained as an embodiment of the present invention. When a substance other than titanium oxide is used as a light-shielding agent, the first coating layer may formulated so as to substantially suppress the production of a substance produced by a reaction of the active ingredient of the present invention, the light-shielding agent used, and light.

For coating of plain tablets, an ordinary coating machine is used. In a coating composition, a coating material may be a main component. Examples of the coating material include hydroxypropylmethylcellulose and the like. In addition, a plasticizer, a light-shielding agent and the like may be added, if necessary. Examples of the plasticizer include macrogol, propylene glycol and the like. Examples of the light-shielding agent include those ordinarily used in manufacture of pharmaceuticals such as titanium oxide and others. In the present invention, a preferred light-shielding agent includes titanium oxide.

A formulation ratio of these ingredients should be chosen so as to sufficiently achieve a purpose of addition of each of the ingredients. For example, a ratio of the coating material : plasticizer : light-shielding agent may be 5 : 1 : 1. For the coating, these ingredients for coating are dissolved or suspended in water or an organic solvent to form a coating liquid, and the coating liquid is sprayed on plain tablets.

A concentration of a solid content of the coating liquid varies depending on characteristics of a coating machine, operating conditions, and quality of an intended coating. The concentration may be, for example, 1 to 50%, preferably 10 to 15%. An amount of the coating per plain tablet needs to be an amount that can sufficiently achieve the object of suppressing the production of the decomposition products of the aforementioned compound, and the amount varies depending on a content of the active ingredient, weight, size, and shape of plain tablets and the like. For example, for an plain tablet containing 0.2 mg of the active ingredient and having a weight of 135 mg and a size 9R of 7mm in diameter, 3 mg or more of the first coating is required to suppress the production of the decomposition product by "the compound + titanium oxide + light", and 3 mg or more of the second coating is required to suppress the production of the decomposition products by "the compound + light".

The pharmaceutical preparation of the present invention is preferably prepared in the form of a tablet. The dose of the pharmaceutical preparation of the present invention may be appropriately determined considering a purpose of administration such as therapeutic treatment or prevention, a type of a disease to be treated or prevented, symptoms, the body weight, age, and sexuality of a patient and the like, and the dose may be usually administered to an adult in an amount of about 0.01 to 1000 mg per day by oral administration. Such a dose may be administered as divided portions once to several times a day.

### (Examples)

The present invention will be more specifically explained by referring to examples. However, the present invention is not limited by these examples. The active ingredient mentioned in the following examples was 5-methyl-2-(1-piperazinyl)benzenesulfonic acid monohydrate, and the compound used was prepared by the method described in Japanese Patent Unexamined Publication (KOKAI) No.(Hei) 9-221479.

### Example 1

### (Determination of decomposition products)

Mixing, granulation, addition and mixing of a lubricant, and compressing were carried out in an ordinary manner to obtain plain tablets with 7 mm in diameter (Formulation 1) so as to contain 0.2 mg of the active ingredient, a suitable amount of mannitol, 23.0 mg of cornstarch, 6.8 mg of low substituted hydroxypropylcellulose, 10.1 mg of hydroxypropylcellulose, and 2.7 mg of magnesium stearate per a plain tablet having a total weight of 135 mg.

Coating was carried out on each plain tablet of Formulation 1 so as to give a coating in a total weight of 5.0 mg including 3.1 mg of hydroxypropylmethylcellulose, 0.6 mg of propylene glycol, 0.9 mg of titanium oxide (CR-EL, henceforth also referred to as the "titanium oxide A") and 0.4 mg of talc, thereby coated tablets (Formulation 2) containing the titanium oxide A were obtained.

The tablets of Formulations 1 and 2 were irradiated with light at 600,000 Lux•hr and 1,200,000 Lux•hr by using a light irradiation-test apparatus, and examination of analogue compounds was carried out by using high performance liquid chromatography (HPLC). For the examination of analogue compounds, the tablets obtained above were extracted with a diluent, which was prepared by dissolving 7.8 g of sodium dihydrogenphosphate dihydrate in 1000 mL of a mixture of water/acetonitrile (6:1) and adjusting its pH to 7.0 with addition of 8 mol/L sodium hydroxide solution, and then the extract was subjected to the HPLC analysis and results were compared with those obtained from an authentic sample. The sample solution was prepared in a darkroom. HPLC conditions were as follows.

### Conditions for the HPLC analysis

Detection: at 220 nm by using ultraviolet absorptiometer
Column: OTS (octylsilylated silica gel) was packed, 4 mm in diameter × 250 mm Column temperature: 40°C
Mobile phase: 7.8 g of sodium dihydrogenphosphate dihydrate was dissolved in 1000 mL of water/acetonitrile mixture (6:1).
Flow rate: adjusted so as to give a retention time of about 7 minutes for the active ingredient.

The test results are as shown in Table 3.

In the plain tablets, the amounts of the substance B and substance C were increased dose dependently with light irradiation, and when a coating containing titanium oxide for light shielding was provided, the amount of the substance A was increased dose dependently with light irradiation. The amounts of the photodecomposition products were in unacceptable ranges, and therefore, a countermeasure for suppressing their productions was necessary.

**Table 3:**

| Results of examination of analogue compounds | | | |
|---|---|---|---|
| Formulation (light irradiation dose (Lux•hr)) | Substance A (R6.4) | Substance B (R15.6) | Substance C (R22.8) |
| Formulation 1 (0) | 0.0 | 0.0 | 0.0 |
| Formulation 1 (600,000) | 0.0 | 0.5 | 0.3 |
| Formulation 1 (1,200,000) | 0.0 | 1.2 | 0.7 |
| Formulation 2 (0) | 0.1 | 0.0 | 0.0 |
| Formulation 2 (600,000) | 0.6 | 0.0 | 0.0 |
| Formulation 2 (1,200,000) | 1.2 | 0.0 | 0.0 |
| (Production ratios (%) of the substances: the numerals after "R" indicate retention times in HPLC analysis) | | | |

### Example 2

### (Confirmation of necessity of two-layer coating)

The plain tablets of Formulation 1 obtained in the above Example 1 were coated so as to give a coating in a total weight of 5.0 mg including 3.8 mg of hydroxypropylmethylcellulose, 0.7 mg of propylene glycol and 0.5 mg of talc, thereby tablets having a coating not containing titanium oxide (Formulation 3) were obtained.

The plain tablets of Formulation 1 were coated so as to give a coating in a total weight of 5.0 mg including 3.1 mg of hydroxypropylmethylcellulose, 0.6 mg of propylene glycol, 0.9 mg of titanium oxide (A-100, henceforth also referred to as the "titanium oxide B") and 0.4 mg of talc, thereby tablets having a coating containing the titanium oxide B (Formulation 4) were obtained.

The plain tablets of Formulation 1 were coated so as to give a coating free from titanium oxide in a total weight of 5.0 mg including 3.8 mg of hydroxypropylmethylcellulose, 0.7 mg of propylene glycol and 0.5 mg of talc as a first coating layer, and further coated so as to give a coating containing the titanium oxide A in a total weight of 5.0 mg including 3.1 mg of hydroxypropylmethylcellulose, 0.6 mg of propylene glycol, 0.9 mg of titanium oxide (CR-EL, the titanium oxide A) and 0.4 mg of talc (coated amount was 10.0 mg in total), thereby two-layer coated tablets (Formulation 5) were obtained.

The tablets of Formulations 1 to 5 were irradiated with light at 3,000,000 Lux·hr by using a light-irradiation test apparatus, and an examination of analogue compounds was carried out by using HPLC. The examination of analogue compounds was performed in the same manner as those described above.

The test results are as shown in Table 4.

The production of decomposition products produced in the plain tablets was not successfully suppressed by the coating not containing titanium oxide. Further, although the coating containing the titanium oxide A or B successfully suppressed the production of decomposition products produced in the plain tablets, other decomposition products were produced. The productions of the decomposition products produced by "the compound + light" and "the compound + titanium oxide + light" were suppressed by the two-layer coating which did not allow contact of the compound with titanium oxide.

**Table 4:**

| Results of examination of analogue compounds after light irradiation of 3,000,000 Lux•hr | | | |
|---|---|---|---|
| Formulation | Substance A (R6.4) | Substance B (R15.6) | Substance C (R22.8) |
| Formulation 1 (plain tablet) | 0.0 | 3.4 | 0.5 |
| Formulation 2 (Titanium oxide A) | 0.5 | 0.0 | 0.0 |
| Formulation 3 (not containing titanium oxide) | 0.0 | 3.5 | 0.6 |
| Formulation 4 (Titanium oxide B) | 0.3 | 0.0 | 0.0 |
| Formulation 5 (two-layer coating) | 0.0 | 0.0 | 0.0 |
| (Production ratios of the substances: the numerals after R indicate retention times in HPLC analysis) | | | |

### Example 3

### (Confirmation of required amount of coating)

The plain tablets of Formulation 1 obtained above were coated with the same composition as that of the first coating of Formulation 5 so as to give a coating amount of 0, 1, 3 or 5 mg, and successively coated with the same composition as that of the second coating of Formulation 5 so as to give a coating amount of 3 mg, thereby two-layer coating tablets of Formulation 6, 7, 8, and 9 were obtained.

The plain tablets of Formulation 1 obtained above were coated with the same composition as that of the first coating of Composition 5 so as to give a coating amount of 3 mg, and successively coated with the same composition as that of the second coating of Composition 5 so as to give a coating amount of 0, 1 or 5 mg to obtain two-layer coating tablets of Formulations 10, 11 and 12.

The tablets of Formulations 6 to 13 were irradiated with light at 1,500,000 Lux•hr by using a light-irradiation test apparatus, and examination of analogue compounds was performed by using HPLC. The examination of analogue compounds was performed in the same manner as that described above.

The test results are as shown in Table 5.

The production of the decomposition products was successfully suppressed by a coating of 3 mg for both of the first and second coatings.

**Table 5:**

| Results of examination of analogue compounds after light irradiation of 1,500,000 Lux•hr | | | |
|---|---|---|---|
| Formulation (coating amount) | Substance A (R6.4) | Substance B (R15.6) | Substance C (R22.8) |
| Formulation 6 (0 mg + 3 mg) | 0.8 | 0.0 | 0.0 |
| Formulation 7 (1 mg + 3 mg) | 0.1 | 0.0 | 0.0 |
| Formulation 8 (3 mg + 3 mg) | 0.0 | 0.0 | 0.0 |
| Formulation 9 (5 mg + 3 mg) | 0.0 | 0.0 | 0.0 |
| Formulation 10 (3 mg + 0 mg) | 0.0 | 3.5 | 0.6 |
| Formulation 11 (3 mg + 1 mg) | 0.0 | 1.0 | 0.3 |
| Formulation 12 (3 mg + 5 mg) | 0.0 | 0.0 | 0.0 |
| (Production ratios of the substances: the numerals after R indicate retention times in HPLC analysis) | | | |

### Industrial Applicability

According to the present invention, a stable pharmaceutical preparation can b be provided which comprises an aminobenzenesulfonic acid derivative known as a therapeutic agent for cardiac insufficiency.

## Claims

1. A pharmaceutical preparation containing an aminobenzenesulfonic acid derivative represented by the following formula (I): 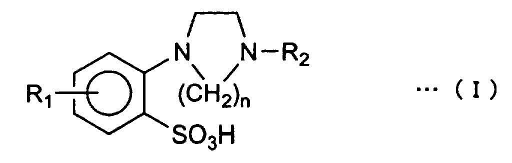 wherein R₁ represents hydrogen atom, a C₁-C₆ alkyl group, a C₃-C₇ cycloalkyl group, a halogenated C₁-C₄ alkyl group, a halogen atom or a C₆-C₁₂ aryl group; R₂ represents hydrogen atom, a C₁-C₆ alkyl group or a C₇-C₁₂ aralkyl group which may have one or more substituents selected from the group consisting of cyano group, nitro group, a C₁-C₆ alkoxyl group, a halogen atom, a C₁-C₆ alkyl group and an amino group; and n represents an integer of 1 to 4 or a salt thereof, or a hydrate thereof or a solvate thereof as an active ingredient, wherein each production of substance A having a retention time of about 6.4 minutes in a high performance liquid chromatography, substance B having a retention time of about 15.6 minutes in the high performance liquid chromatography, and substance C having about 22.8 minutes in the high performance liquid chromatography is substantially suppressed, wherein said high performance liquid chromatography is performed at a controlled flow rate for elution so as to give a retention time of about 7 minutes of said active ingredient by using an ultraviolet absorptiometer at 220 nm, an octylsilylated silica gel packed column (4 mm x 250 mm) at 40°C, and a mobile phase prepared by dissolving 7.8 g of sodium dihydrogenphosphate dihydrate in 1000 mL of a mixture of water/acetonitrile (6:1) after said pharmaceutical preparation is extracted with a diluent prepared by dissolving 7.8 g of sodium dihydrogenphosphate dihydrate in 1000 mL of a mixture of water/acetonitrile (6:1) and adjusting a pH to 7.0 with addition of 8 mol/L sodium hydroxide solution.

2. The pharmaceutical preparation according to claim 1, wherein the coating layer consists of two layers.

3. The pharmaceutical preparation according to claim 1 or 2, wherein the coating layer consists of a first coating layer for substantially suppressing production of the substance A according to claim 1, and a second coating layer for substantially suppressing production of the substances B and C according to claim 1.

4. The pharmaceutical preparation according to any one of claims 1 to 3, wherein the first coating layer does not contain titanium oxide.

5. The pharmaceutical preparation according to any one of claims 1 to 4, wherein the second coating layer contains titanium oxide.

6. The pharmaceutical preparation according to any one of claims 1 to 5, which is in the form of a tablet.

7. The pharmaceutical preparation according to claim 6, wherein an plain tablet is coated with the first coating layer and then coated with the second coating layer.

8. The pharmaceutical preparation according to any one of claims 1 to 7, wherein the substitution of R₁ is in the 5-position.

9. The pharmaceutical preparation according to any one of claims 1 to 8, wherein n is 2.

10. The pharmaceutical preparation according to any one of claims 1 to 9, wherein R₂ is hydrogen atom, a C₁-C₃ alkyl group or a C₇-C₁₂ aralkyl group which may have one or more substituents selected from a C₁-C₃ alkyl group, a C₁-C₃ alkoxyl group and a halogen atom.

11. The pharmaceutical preparation according to any one of claims 1 to 10, wherein R₂ is hydrogen atom or a C₇-C₁₂ aralkyl group which may have one or more substituents selected from C₁-C₃ alkoxyl groups.

12. The pharmaceutical preparation according to any one of claims 1 to 11, wherein R₂ is hydrogen atom.

13. The pharmaceutical preparation according to any one of claims 1 to 12, wherein R₁ is hydrogen atom, a C₁-C₆ alkyl group, a C₅-C₆ cycloalkyl group, trifluoromethyl group, a halogen atom or phenyl group

14. The pharmaceutical preparation according to any one of claims 1 to 13, wherein R₁ is a C₁-C₃ alkyl group, cyclohexyl group, trifluoromethyl group, chlorine atom, bromine atom or phenyl group.

15. The pharmaceutical preparation according to any one of claims 1 to 14, wherein R₁ is methyl group or propyl group.

16. The pharmaceutical preparation according to any one of claims 1 to 7, wherein the active ingredient is selected from the following compounds:
5-methyl-2-(1-piperazinyl)benzenesulfonic acid;
5-trifluoromethyl-2-(1-piperazinyl)benzenesulfonic acid;
5-n-propyl-2-(1-piperazinyl)benzenesulfonic acid;
5-phenyl-2-(1-piperazinyl)benzenesulfonic acid;
5-chloro-2-(1-piperazinyl)benzenesulfonic acid;
5-bromo-2-(1-piperazinyl)benzenesulfonic acid;
5-iso-propyl-2-(1-piperazinyl)benzenesulfonic acid;
5-cyclohexyl-2-(1-piperazinyl)benzenesulfonic acid;
5-n-propyl-2-(1-homopiperazinyl)benzenesulfonic acid;
5-n-propyl-2-[4-(2,3,4-trimethoxybenzyl)-1-piperazinyl]benzenesulfonic acid;
5-n-propyl-2-[4-(3,4-dimethoxybenzyl)-1-piperazinyl]benzenesulfonic acid.

17. The pharmaceutical preparation according to claim 16, wherein the active ingredient is selected from the following compounds:
5-methyl-2-(1-piperazinyl)benzenesulfonic acid;
5-n-propyl-2-(1-piperazinyl)benzenesulfonic acid.

18. The pharmaceutical preparation according to any one of claims 1 to 17, wherein the active ingredient is 5-methyl-2-(1-piperazinyl)benzenesulfonic acid monohydrate.

19. A tablet wherein a plain tablet containing an aminobenzenesulfonic acid derivative represented by the following formula (I): 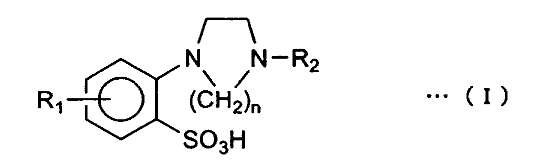 wherein R₁ represents hydrogen atom, a C₁-C₆ alkyl group, a C₃-C₇ cycloalkyl group, a halogenated C₁-C₄ alkyl group, a halogen atom or a C₆-C₁₂ aryl group; R₂ represents hydrogen atom, a C₁-C₆ alkyl group or a C₇-C₁₂ aralkyl group which may have one or more substituents selected from the group consisting of cyano group, nitro group, a C₁-C₆ alkoxyl group, a halogen atom, a C₁-C₆ alkyl group and an amino group; and n represents an integer of 1 to 4, or a salt thereof, or a hydrate thereof or solvate thereof as an active ingredient, is applied with two coating layers, which is characterized to comprise a first coating layer having a property of substantially suppressing production of a substance produced from a reaction of said active ingredient, titanium oxide, and light, and a second coating layer having a property of substantially suppressing production of a substance produced from a reaction of said active ingredient and light.

20. The tablet according to claim 19, wherein the first coating layer does not contain titanium oxide.

21. The tablet according to claim 19 or 20, wherein the second coating layer contains titanium oxide.

22. The tablet according to any one of claims 19 to 21, wherein the plain tablet is coated with the first coating layer and then coated with the second coating layer.
